# EUROPEAN PATENT SPECIFICATION

(11) **EP 2 578 206 B1**
(45) Date of publication and mention of the grant of the patent: **09.12.2015**
(21) Application number: 11790065.4
(22) Date of filing: 12.04.2011
(51) Int. Cl.: A61K 9/02, A61K 31/198, A61K 33/04, A61K 33/30, A61P 13/00

(54) **PHARMACEUTICAL COMPOSITION FOR THE TREATMENT OF DISORDERS OF THE LOWER SECTION OF THE UROGENITAL SYSTEM**
PHARMAZEUTISCHE ZUSAMMENSETZUNG ZUR BEHANDLUNG VON STÖRUNGEN DES UNTEREN TEILS DES UROGENITALSYSTEMS
COMPOSITION PHARMACEUTIQUE POUR TRAITER LES AFFECTIONS DE LA PARTIE INFÉRIEURE DU SYSTÈME UROGÉNITAL

(30) Priority: 03.06.2010 RU 2010123729
(43) Date of publication of application: 10.04.2013
(73) Proprietor: Obshestvo S Ogranichennoj Otvetstvennostju "CytoNIR", St. Petersburg 191023 (RU)
(72) Inventor: MALININ, Vladimir Victorovich, St.Petersburg 197227 (RU)
(74) Representative: Jeck, Anton
(86) International application number: PCT/RU2011/000240
(87) International publication number: WO 2011/152755

(56) References cited:
- DE-A1- 2 438 703
- JP-A- 61 065 822
- RU-C2- 2 264 819
- RU-C2- 2 356 535
- US-A- 4 258 037
- US-B1- 6 197 309
- DATABASE WPI Week 198620 Thomson Scientific, London, GB; AN 1986-128195 XP002715082, & JP S61 65822 A (KAO CORP) 4 April 1986 (1986-04-04)
- APOLIKHIN O.I. ET AL.: 'Znachenie preparatov selena i tsinka dlya organov mochepolovoy sistemi.' CONSILIUM MEDICUM, [Online] vol. 10, no. 4, 2008, pages 118 - 119 Retrieved from the Internet: <URL:http://doctor-urolog.narod.ru/interest ing/ sezn/sezn.htm> [retrieved on 2011-07-14]
- FEINBLATT H. M. ET AL.: 'Palliative Treatment of Benign Prostatic Hypertrophy.' THE JOURNAL OF THE MAINE MEDICAL ASSOCIATION, [Online] vol. 49, no. 3, March 1958, pages 99 - 101 Retrieved from the Internet: <URL:http://www.prostex.com/feinblatt-bph-a rticle> [retrieved on 2011-08-19]

## Description

### Field Of The Invention

The claimed invention relates to medicine, specifically, to a medicinal preparation, namely suppositories comprising a medication for the treatment of lower genitourinary system diseases. This invention can be widely used in medical practice for treating urological and gynecological diseases.

### Background of the invention

The medication in suppository form, used for the treatment of chronic prostatitis, comprises 4 - 6 mg of a bioactive compound from bovine prostate concentrate per each suppository weighing 2.0 - 2.2 g and also, a purified liquid α-interferon concentrate or a recombinant interferon [RU # 2160114, M.cl.⁶ A61K 35/48, 2000]. The suppositories are gelatin-, glycerin-, and sodium carbonate-bicarbonate buffer-based. Interferon provides antiviral protection, thus, the suppositories may be used according to RU # 2160114 when treating chronic prostatitis in immunocompromised patients.

However, the efficacy of said suppositories is low, which could be attributed to the low concentration of the bioactive compound and interferon's instability in this composition.

The pharmaceutical composition in suppository form comprises 0.05 - 0.40 g of the bioactive compound and 0.7 g of the antimicrobial agent per each suppository weighing 2.15 - 2.35 g [RU # 2304979, M.cl.⁷ 61 K 38/02, 2007]. The composition contains a bovine prostate bioregulatory peptide complex as a bioactive compound. The composition also contains antibiotics of the fluoroquinolone, penicillin, cephalosporin, or tetracycline, etc. groups as antimicrobial agents. Suppositories according to RU # 2304979 could also comprise antiviral agents and antiprotozoal drugs. They were tested on a prostatitis model of white male rats.

Antibiotics in suppositories according to RU # 2304979 may cause the usual complications expressed in humans i.e., allergic reactions to antibiotics as well as lowered immunity usually caused by antibiotics.

Pharmaceutical compositions according to RU # 2160114 and RU # 2304979 have one common disadvantage - the use of the bioregulatory peptide complex as a bioactive compound.

Typically for any naturally derived products, their structure is not fully identified since the methods used for their identification and standardization are complicated and also, because they are not always available to both the manufacturer and the researcher. The content of the active ingredients does not exceed 20%. A naturally derived preparation always contains a number of high-molecular-weight compounds inseparable from the active ingredients.

All this narrows the spectrum of the drug's activity.

### Disclosure of the invention

The invention is directed to broadening the spectrum of activity of the pharmaceutical composition.

The invention is also directed to the departure from the use of natural materials.

The stated objective is achieved by making a pharmaceutical composition in suppository form, said composition comprising a bioactive compound and a fatty base, wherein the bioactive compound is a mixture of amino acids comprising glutamic acid, lysine, alanine, arginine and glycine and further comprising zinc in the form of zinc chloride in the following component ratio expressed in grams per one suppository weighing 2.7 - 2.9 g each:

| | |
|---|---|
| Glutamic acid | 0.050 - 0.500 |
| Lysine | 0.025 - 0.250 |
| Alanine | 0.025 - 0.250 |
| Arginine | 0.025 - 0.250 |
| Glycine | 0.025 - 0.250 |
| Zinc | 0.010 - 0.150 |
| Fatty base | remaining |

The pharmaceutical composition may further comprise 10 - 30 mcg of selenium as anhydrous sodium selenite or 0.020 - 0.035 g of sodium fumarate per suppository.

Different amino acids are known to regulate different physiological functions of living organisms [Chirkin A.A., Danchenko Ye.O., Biochemistry. M., "Meditsinskaya literatura" (Medical Literature), 2010, 608 p.] Thus, there are medications in suppository form that are used for the treatment of female sexual dysfunctions [RU application # 2000120537, M.cl.⁷ A61K 45/06, 2002] comprising 2 - 10 mg of the α-adrenergic receptors antagonist (Yohimbine, hentolamine, etc.) and 1 - 8 g of arginine exhibiting NO-donor activity.

Also known in the art are the anti-herpes medications, for example, in suppository form [RU #2264819, M.cl.⁷ A61K 35/74, 2005 and RU application # 2004128635, M.cl.⁷ A61K 31/19, 2006] comprising anti-herpes virion vaccine and polyoxidonium immunomodulator as well as valine, lysine, isolysine and a combination of at least two amino acids selected from the group including but not limited to alanine and arginine.

Also known in the art is a pharmaceutical composition, for example, in suppository form, for treatment of erectile dysfunction [RU application #2000100301, M.cl.7 A61K 31/475, 2001] comprising 2 - 8 mg of Yohimbine and 1 -4 g of arginine.

Also known in the art is are anti-rheumatic medications in the form of suppositories comprising a mixture of 15 amino acids with pharmaceutically acceptable additives, selected from arnica extract, sea salt, lactate, vitamin B2 and B6 and Senna extract (DE 2438703).

US 4258037 describes a suppository composition comprised of a combination of an extract of plants of the Rosacea family with the three amino acids glycine, L-glutamic acid and L-alanine for use in the treatment of urogenital disorders such as prostatic disorders and bartholinitis. US 6197309 discloses a pharmaceutical composition containing zinc, glycine, alanine, glutamic acid and selenium for the treatment of the prostate gland. Feinblatt H.M. et al.( "Palliative treatment of benign prostatic hypertrophy", The Journal of the Maine Medical Association, vol. 49, no.3, march 1958, pages 99-101) describes a capsule composition consisting of glycine alanine and glutamic acid for treating benign prostatic hypertrophy.

The underlying technical problem solved by the present application therefore is to provide an alternative pharmaceutical composition for the treatment of both male and female urogenital disorders in suppository form.

It is known in the art that when different biologically active compounds are combined in a pharmaceutical composition, their cumulative beneficial effect and even their compatibility are unpredictable [see, for example, Kochetygov N.I. "Blood substitutes after blood loss or shock". L., "Meditsina", 1984, p. 148; edited by Oleynik S.A. "Sports pharmacology and dietology". M. OOO I.D. Williams, 2008, 256 p.; "Fundamental of clinical nutrition. Course lectures for the European Association of Parenteral and Enteral Nutrition". M., ItelTec, 2003, 416 p.]

Thus, the physiological activity of the claimed composition in the treatment of prostate diseases (prostatitis, prostate adenoma, etc.) as well as in the treatment of gynecological diseases, such as vaginitis, was unexpected and unpredictable. All components of the claimed composition are commercially available. The following components were used: glutamic acid (Ajinomoto, Japan), lysine (Sigma-Aldrich, Germany), alanine (Merck, Germany), arginine (Ajinomoto, Japan), glycine (Jizhou City Huayang Chemical, China), and zinc chloride (Merck, Germany); its content in the claims is given calculated for zinc.

When the composition contains 10 - 30 mcg of selenium, it is present in the anhydrous sodium selenite form.

The composition may also contain 0.020 - 0.0035 g of sodium fumarate.

The base could be made of but not limited to cocoa butter, edible fats, a mixture of mono-, di, and fatty acid triglycerides (Witepsol) and other fatty bases listed in the pharmacopeia.

The suppositories are prepared as follows:

### Example 1. Preparation of the suppositories

### 1. Preparation of the suppository mass.

### 1.1 Preparation of the premix.

The premix is prepared in a homogenizer with a heatable jacket.

To prepare the premix, a sufficient amount of suppository base (Witepsol/W35) is placed into a homogenizer and melted.

The premeasured amounts of glutamic acid, alanine, lysine, arginine, glycine, zinc, and also, possibly, sodium fumarate and selenium are added. The premix is homogenized for 20 - 30 min. The premix is checked for quality upon completion of the process: the mass must be homogeneous.

### 1.2 Mixing the premix with the base

The suppository base ((Witepsol/W35) is placed in a reactor and melted. The premix is added to the base and the resulting mixture is stirred for 1 hr. The suppository mass must be homogeneous with no visible inclusions.

### 2. Making the suppositories

### 2.1 Filling blister molds with the suppository mass (filling)

Blister molds are filled with a dispenser. The suppository mass is transferred into the dispenser's hopper. The molds are filled with the suppository mass at a constant temperature with stirring.

The blister mold filled with the suppository mass is rolled with guide rollers and passed onto the "Suppository chilling" step.

### 2.2 Chilling the suppositories

The suppositories are cooled in a refrigerator at 10 - 15°C for 1 - 2 hrs.

Acute and chronic toxicity of the claimed suppositories was examined as per the requirements of the "Guide to the Experimental (Preclinical) Study of Novel Pharmaceutical Substances (2005)." Acute toxicity was studied on 36 outbred white female mice. Chronic toxicity was studied on 45 outbred white female mice and 9 male rabbits.

In the acute toxicity study, the preparation was administered in increasing doses of 1.0, 2.0, 4.0, 8.0 and 10.0 g/kg (per finished dosage form) as per Litchfield-Wilcoxon, based on an average suppository weight of 2.0 g.

A single administration of the preparation of the present invention to animals in doses exceeding the clinically recommended therapeutic dose by more than 500 times was not shown to cause any toxic reactions, which validates the wide therapeutic application range of the preparation.

A chronic toxicity study of the preparation showed no side effects following the use of the suppositories over a period of 30 days in doses exceeding therapeutically recommended doses by more than 200 times. The study did not yield any credible evidence of the effect of the preparation of the present invention on the morphological and biochemical peripheral blood indicators, on the ESR, and the erythrocyte resistance. Additionally, total protein in blood serum was determined by Lowry Protein Assay, while potassium and sodium were determined by plasma spectrophotometry. A pathomorphological study of the brain, spinal fluid, spinal ganglions, thyroid gland, parathyroid glands, adrenal glands, testicles, pituitary glands, heart, lungs, aorta, liver, kidneys, bladder, pancreas, stomach, small intestine, large intestine, thymus, spleen, lymph nodes and bone marrow was conducted upon completion of the experiment.

No pathologies were detected in the animals when evaluating the overall condition thereof, the morphological and biochemical indicators of peripheral blood, the morphology of internal organs, the condition of the cardiovascular and respiratory systems, and liver and kidney function.

Due to the absence of general toxicity, the suppositories may be recommended for testing in vivo in experimental genitourinary pathology. The pharmacological effect of the claimed composition was studied on the experimental models of prostatitis and vaginitis.

### Example 2. Efficacy of the suppositories on a model of irritant prostatitis in rats

The experiments were conducted on outbred male rats weighing 180 - 200 g, raised in the RAMS nursery "Rappolovo." Before the experiments, the animals were quarantined for 14 days and randomized. Marks and identity numbers of the animals were registered in the laboratory test protocols. The animals were kept in a vivarium on a standard diet.

Chronic prostatitis was simulated by administering a 10% aqueous solution of dimexidum mixed with turpentine to all the experimental animals in a 4:1 volumetric ratio. Prior to the administration, the mixture was shaken for 1 min until a fine emulsion had occurred. The freshly obtained emulsion was administered to the rats rectally, 20 - 25 mm deep in a 1 ml volume with a rectal infusion device (dispenser) ensuring a trauma-free procedure. The animals were immobilized in a horizontal position with canvas gloved hands. Prior to the administration, the mixture was shaken until formation of a fine emulsion.

Turpentine in said rectal administration mixture is used to produce pathological hyperemia in the prostate, while the aqueous dimexidum solution is a conductor facilitating turpentine's penetration through the tissues and acting as an analgesic.

The preparations of the present invention were administered to rats in different groups individually; 28 days post pathology stimulation in the doses below. The animals were divided into the following groups, 10 rats each:
- intact animals
- control 1 (irritant prostatitis)
- control 2 (irritant prostatitis + comparator drug Prostatilen (bioregulatory peptides from bovine prostate) 190 mg/kg p.r.)
- Irritant prostatitis + claimed suppositories, 240 mg/kg p.r.

The preparations of the present invention were administered to each male rat individually in a molten state with an atraumatic probe.

The reduction in urodynamics was measured (by indicators of a spontaneous 24-hr diuresis) with individual exchange cells from Techniplast (Italy). Baseline diuresis indicators (28 days post pathology stimulation) and post therapy indicators (58 - 59 days) were determined.

The 24-hr urine level was tested for excreted protein by quantitative turbidi-metric assay.

Upon collecting urine in exchange cells, the rats were instantly decapitated under light halothane anesthesia. Blood samples were collected into vacuum clot activator tubes Vacutest SER (Vacutestkima, Italy). The samples were heated for 30 min at 37°C in a thermostat, cooled for 1 hr. in a refrigerator, and centrifuged at 2,050 rpm. Blood serum was separated into Microtubes Eppendorf test tubes (Plastibrand, Germany.)

Blood serum was tested for the testosterone level by IFA kits (XEMA); nitrogen oxide by Griess photometric assay at 520 nm; thiobarbiturate-reactive products (ma-Ionic dialdehyde) by a photometric assay at 537 nm; C-reactive protein by IFA (BD kits); ceruloplasmin levels by Ravin's photometric assay; prostatic acid phosphatase activity by kinetic photometric assay with Olvex Diagnosticum kit.

For histological assay, the ventral prostate was immobilized in a 10% neutral formalin solution and, upon a standard passage through a series of solvents, poured into paraffin. Tissue slices 5 - 7 mcm thick were stained with hematoxylin-eosin.

The following 5-point scale was used to compare the severity of prostatitis in different groups:
1 point-traces of prostatitis (small foci of fibrosis, interstitial lymphocytic and histiocytic infiltration);
2 points - foci of chronic prostatitis with inflammatory infiltration (mainly interstitial) covering less than % of the gland slice;
3 points - foci of prostatitis with large inflammatory infiltrates around the ducts, acini and blood vessels, with leucocytes accumulated in glandular lumens covering ¼-1/2 of the gland slice;
4 points - severe prostatitis covering ¼ - ¾ of the gland slice;
5 points - acute prostatitis covering more than % of the gland slice.

Statistical data were processed using a standard Microsoft Excel program package. Substantial changes were calculated using Student's t-test.

Experimental prostatitis simulation resulted in significant pathological urodynamic changes in rats 28 days post phlogogen (a mixture of turpentine and dimexidum) administration. Administration of the turpentine and dimexidum mixture resulted in prostatitis, causing a reduction in urodynamics on account of an inflammatory increase in the weight/volume gland indicators. In all experimental groups, a 24-hr diuresis 28 days post simulation (pathology baseline) was reduced by more than 40%. Proteinuria increased significantly (5.4 times), which is attributed to the outpouring of inflammatory exudate into the urethral lumen of the male rats, and its components contaminating the urine. An increased output of acid phosphatase isoenzyme, the main marker of prostatitis and adenoma, from the tissue occurred at the pathology baseline. The acute-phase protein level was observed to increase 24 times for ceruloplasmin and 2.2 times for C-reactive protein, while the testosterone level decreased.

The experimental prostatitis model results in an inflammatory increase of the weight/volume dimensions of the ventral prostate, which was assessed at the end of the experiment based on the gland's weight while evaluating the effect of the medications on the course of the disease.

The gland's enlargement was largely caused by edema and hyperemia of the gland tissue. These changes play a significant role in the impairment of urodynamics during the passage of urine, they are the main factor in the urinary bladder wall hyperextension in patients suffering from prostatitis and prostate adenoma as well as a cause of dysuric disorders, including pain during urination.

Therapy with the preparations of the present invention conducted over a one-month period resulted in a significant weight normalization of male rats with experimental prostatitis. Prostatilen decreased the ventral prostate weight by 23.7% as compared to the control receiving no treatment. The suppositories of the present invention were somewhat more instrumental in the normalization of this indicator- a 30.4% reduction.

Administration of the comparator drug Prostatilen significantly reduced proteinuria in rats (3.2 times) and led to a 54.3% restoration of diuresis as compared to the pathology baseline.

Administration of suppositories of the present invention facilitated the restoration of urodynamics, yielding a 22.4% increase in diuresis; and the reduction of daily spilling of protein into urine (proteinuria) by 2.2 times compared to the pathology baseline in this group.

Therapy with the comparator drug Prostatilen reduced the intensity of the oxidative processes in the gland, which was evident from the considerable reduction in both nitrogen oxide levels (1.5 times) and the levels of the products reacting with thiobarbituric acid (TBA-RP) (2.2 times) compared to control animals not receiving treatment.

Suppositories of the present invention, when administered to rats, had a effect comparable to Prostatilen on the intensity of oxidative stress: nitrogen oxide was reduced 1.8 times, and TBA-RP was reduced 1.9 times as compared to control.

Therapy with Prostatilen was found to credibly reduce acid phosphatase isoenzyme output (1.7 times) and to increase the testosterone level (1.6 times) in comparison with the pathology control.

Suppositories of the present invention had a significantly stronger effect on the activity of prostatic acid phosphatase (2.4 times reduction as compared to control and 1.4 times reduction as compared to the comparator drug) and the testosterone level (1.4 times increase.)

Prostatilen reduced the acute-phase protein blood levels of ceruloplasmin 2.4 times and of C-reactive protein 1.8 times. Therapy with suppositories of the present invention also helped normalize the ceruloplasmin level (2.5 times) and, especially, the level of C-reactive protein (2.2 times).

A morphological study of the slices demonstrated that therapy with the comparator drug Prostatilen reduced the intensity of the inflammatory infiltration of the gland's stroma and white blood cells detectable in the acini. Signs of inflammation in histological preparations were observed in only 40% of the male rats used in the study. The rest of the animals were found to have normal gland tissue structures.

The effect of the suppositories of the present invention was similar to that of Prostatilen. Foci of chronic prostatitis with inflammatory infiltration (mostly interstitial) covering more than ¼ of the gland slice occurred in only 40% of the animals. The prostate gland structure in the rest of the rats corresponded to that of the intact animals.

All in all, the claimed pharmaceutical composition has a similar or better therapeutic effect on the experimental prostatitis as Prostatilen.

### Example 3. Efficacy of the suppositories on a model of irritant vaginitis in rats

The experiments were conducted on white outbred female rats weighing 180 - 200 g

Vaginitis was stimulated by a one-time intravaginal application of an irritant, a mixture of oil of turpentine and dimexidum in a 1:1 ratio in the dose of 0.5 ml per 100 g of the animal's weight.

The animals from the experimental group were treated 24 hours post administration of the irritant. The preparation was administered vaginally over a 7-day period in doses corresponding to the daily human dose. The daily dosage form recalculated for rats was 220 mg/kg. The preparation was administered once a day. To prevent the suppositories from free evacuation, the vaginal opening was plugged with a small cotton tampon for 30 min.

The animals of the control group did not receive treatment. They were followed for the dynamics of the inflammatory process by evaluating the cell composition of vaginal smears and by a microbiological study of the impression smears at the onset and on the 1st, 3rd, and 7th day post application of the irritant. The hematologic markers of the inflammatory process: white blood cell content in the peripheral blood, white blood cell differential, and the ESR were evaluated at the same times. The animals' behavior and overall condition were also followed. The following factors were also taken into consideration:
- Intensity of the local hyperemia expressed in points: 0 - visible vaginal mucosa and skin around the vagina are unchanged; 1 - slight hyperemia of the visible vaginal mucosa; 2 points - pronounced hyperemia of the vaginal mucosa and the skin; 3 - pronounced hyperemia of the vaginal mucosa and visible signs of self-inflicted wounds - bite marks.
- Intensity of the vaginal discharge expressed in points: 0 -no discharge; 1-light discharge when pressed with a swab; 2 - moderate discharge, the skin around the vagina is wet; 3 - heavy discharge, the skin around the vagina and the bedding are wet.

Histological control was carried out on the 3^{rd}, 7^{th}, and 10^{th} day. For the histological study, the rats from the experimental and control groups were euthanized by decapitation under light ether anesthesia.

The vagina was fixed in 10% formalin and after alcohol prep, it was poured into paraffin. The slices were stained with hematoxylin-eosin and examined under an MBI-6 biological microscope.

All the animals in all experimental groups were found to have severe anxiety, vaginal discharge, and hyperemia of the visible vaginal mucous membrane 24 hrs. post administration of the irritant.

The first day post stimulation of vaginitis, the control group showed anxiety and a hypersensitivity to external irritants. They were observed to have reduced food and water intake and ruffled fur. All the animals were found to have seromucous vaginal discharge. By the third day of the experiment, behavioral deviations of the animals were gradually diminished. On the 7^{th} day, these rats' behavior was no different from the behavior of the intact (not taking part in the experiment) rats.

The condition and behavior of the animals receiving suppositories of the present invention on the first day of the experiment was no different from the control group. They were observed to have ruffled fur, increased anxiety, and aggressive response to handling. Administration of the preparation was accompanied by anxiety and vocalization. Reduced vaginal discharge and hyperemia were observed on the 3^{rd} day post administration of vaginal suppositories. These changes were statistically significant compared to control. On the 7^{th} day of the experiment, all visible symptoms of inflammation completely disappeared.

On the first day after the start of therapy, all experimental groups showed signs of acute inflammation with severe leukocytosis and a white blood cell differential shift to the left, to the formation of stab white blood cells, lymphocytopenia. High ESR was also noted.

A positive result was observed already on the first day of therapy with the suppositories of the present invention. The animals receiving treatment had their ESR reduced 1.3 times; the number of leucocytes also decreased. This trend was observed throughout the entire experiment.

On the 3^{rd} day, while the animals of the control group showed no signs of positive dynamics, the animals receiving suppositories of the present invention showed a significant decrease in the number of leukocytes and segmented neutrophils as well as reduced intensity of lymphocytopenia compared to the first day after the start of therapy.

On the 7^{th} day, the control group (receiving no treatment) began showing some positive dynamics in the studied parameters. The group of animals receiving suppositories of the present invention showed a significant decrease in the total white blood cell count, a slight stab shift, and the restored number of neutrophils and lymphocytes. The ESR on the 7^{th} day was no different from the ESR of the intact animals.

Introduction of the irritant adversely affected vaginal microflora of the animals in all experimental groups. Throughout the experiment, the control group showed significant changes confirming an inflammatory process: a high content of vegetative and nonvegetative yeast forms and an increased amount of diplococcal and coccal flora in the smears. A trend towards normalization of vaginal microflora was observed only on the seventh day.

Treatment with the suppositories of the present invention resulted in a decreased number of diplococci, yeast, gram-negative bacilli and gram-positive cocci already on the 1^{st} day. On the third day, the treatment of animals with the suppositories of the present invention resulted in restoration of the natural microflora composition (its qualitative and quantitative characteristics) to a level practically identical to the norm. The presence of an increased amount of lactobacteria with high antagonistic activity toward a wide range of pathogenic and opportunistic pathogenic microbes must also be noted; this defines the corrective effect of the preparation on the impaired bacterial biocenosis.

A 7-day treatment with the preparation reduced the pathogenic flora in the smears and restored vaginal microbial biocenosis. Gram-positive and Gram-negative cocci, Gram-negative bacteria and both vegetative and nonvegetative yeast were all sensitive to the effect of the suppositories of the present invention.

Thus, the irritant vaginitis model demonstrated a strong antimicrobial effect of the suppositories of the present invention. Treatment with the preparation resulted in restoration of the natural balance of vaginal microorganisms.

A morphological study of vaginal slices of rats with irritant vaginitis was conducted on the 1^{st}, 3^{rd}, and 7^{th} day of the experiment.

In the first three days of the experiment, the control rats were found to have destructive inflammatory changes in all layers of the vaginal walls. The outer layer of the mucous membrane epithelium was in a state of necrobiosis and was entirely missing in some spots. Hyperchromatism of the nuclear membrane and chromatin lysis were observed. Polymorphonuclear leukocytes infiltrated the submucosal layer and penetrated the epithelium. Hyperemia and edema of the loose connective tissue were observed in the deeper strata of the submucosal layer. On the third day, the destructive processes continued. A strong inflammatory reaction was expressed as a massive infiltration with granular leukocytes and the emergence of macrophages, lymphocytes and plasmatic cells. By the 7^{th} day of the experiment, the changes in vaginal mucous membrane were not very pronounced: the epithelium of the mucous membrane was fully regenerated, the nuclei of the epithelium cells were clearly defined; hyperemia, edema, and leukocytic infiltration of the submucosal layer were not very pronounced.

Treatment with the suppositories of the present invention in the first three days slightly reduced the intensity of the inflammation of the mucous membrane epithelium. Cell necrobiosis was observed in the outer layers of the epithelium, polymorphonuclear leukocytes infiltrated the inflammation zone, and isolated polymorphonuclear leukocytes, leukocytes, and plasmatic cells were detected.

Treatment with the suppositories of the present invention for 7 days promoted a full restoration of the epithelium integrity; the histological structure of the vaginal mucous membrane did not differ from that of the intact rats.

Data from histological study fully correlated with the results of the hematologic and microbiologic inflammation markers.

Thus, the claimed composition is effective in the treatment of experimental vaginitis.

Clinical efficacy of the suppositories was determined while administering them to male and female patients suffering from diseases of the lower genitourinary system.

### Example 4. Efficacy of the suppositories in treating patients suffering from chronic prostatitis

A study of clinical efficacy of the suppositories was conducted on 22 patients aged 25-47 suffering from chronic prostatitis.

Chronic prostatitis mainly presented as gnawing pain in a typical location, various urination disorders, sexual dysfunctions, and signs of neuroticism.

All the patients earlier received traditional symptomatic and pathogenic drugs providing short-term therapeutic effects and requiring patients to receive increased doses of the preparations in the course of treatment over a long period of time. The suppositories were administered rectally, at bedtime, over a 10 - 15 day period depending on the degree of severity of the disorder. The control group consisted of 10 analogous patients receiving traditional treatment.

The efficacy of the treatment with the claimed suppositories was evaluated based on the dynamics of patients' complaints, complete blood count and urinalysis, biochemical blood profile, and a coagulation profile test pre and post treatment. Abdominal pressure during urination and the character of the urinary stream was expressed in points from 1 to 5 (with 1 being normal and 5 - having the most changes.) Peak and mean urine flow rates, time length of urination, time to reach peak urine flow rate, and the flowmetric index were measured. Along with the aforementioned studies, the prostate gland was palpated, its secretion was analyzed in the laboratory, and an in-depth study of the copulative function was performed. An ultrasonic examination of the prostate gland was also carried out.

When evaluating the results obtained in the treatment of chronic prostatitis, the clinical criteria received the most attention. Upon completion of the treatment, the pain completely disappeared in 72.4% of the patients and considerably diminished in 23.7% of the patients who had reported such complaints. 52.6% of the patients suffering from sexual dysfunction showed a complete recovery and 43.7% reported improvement. The suppositories had positive effects on improved erectile function, enhanced orgasm, and abolition of pain during orgasms. A longer duration of sexual intercourse was also reported. By the end of the treatment, 50.6% of the patients reported a restored libido.

85.4% of the patients no longer complained of pollakiuria (frequent urination). The need for nocturnal urination had disappeared. 82.7% of patients reported no more strangury (painful urination) and 22.9% had a significant improvement in the strength of the urinary stream and easier urination.

Urologic flowgrams recorded after the treatment in patients suffering from prostatitis stage I and II showed the main urination parameters restored to their normal values. On stage III of the disease, recovery was hindered by the reduced elasticity of the urinary bladder neck due to sclerotic changes in the prostate gland tissue, but these patients also reported a significantly stronger urinary stream. When the prostate gland was palpated through the rectum after the treatment with the claimed suppositories, it showed a trend to recover its size and consistency. Additionally, the areas of induration had disappeared and the examination itself was not painful. The observed reduction in the size of the prostate gland could be attributed to the reduced edema of the intestinal tissue and indicates the absence of inflammatory activity within, which was also confirmed by ultrasound diagnostics. The complete clinical and biochemical blood profile tests conducted in the process did not reveal any significant deviations from the norm. No side effects were reported.

Due to the restoration of the prostate function by the suppositories, its secretion quality had improved, which prompted a 23.7% increase in the active sperm count of the ejaculate. The reduced activity of the inflammation process was confirmed by a lowered number of leukocytes in the ejaculate, prostate secretion, and urine. At the same time, the amount of exfoliated epithelial cells in the material under study was found to be lower.

### Example 5. Efficacy of the suppositories in treating older and elderly patients suffering from dysuric disorders

49 patients, including 29 men aged 68 to 82, and 20 women aged 58 to 84, suffering from urination disorders participated in the study. All older and elderly patients complained of frequent urination (10 - 20 times per 24 hrs), especially at night (up to 5 - 10 times), which considerably affected their quality of life and impeded their social adaptation.

The efficacy of treatment was evaluated based on the dynamics of their complaints and urodynamic indicators. Peak and mean urine flow rates, time length of urination, time to reach peak urine flow rate, and the flowmetric index were measured.

The patients were randomized into 2 groups. The control group consisted of 18 people (11 men and 7 women) who receive traditional treatment including spasmolytic preparations (Spasmex, Spasmol, Spasmoveralgin Neo) and cholinolytics (Detrusitol).

31 patients in the main group (18 men and 13 women) were subdivided into 3 subgroups depending on the severity of their urination disorders. The patients with most severe dysuric disorders expressed as frequent urination up to 15 - 20 times per 24 hrs, and especially at night (up to 10 times), were rectally receiving the suppositories in the morning and at bedtime over 15 days.

The patients with moderate urination disorders expressed as frequent urination (up to 10 times per 24 hrs. including up to 5 times at night) were rectally receiving the suppositories at bedtime over 10 - 15 days.

A comparison of the indicators for patients in the control and main groups showed pronounced positive dynamics in the group of patients receiving the suppositories.

All the patients receiving the suppositories assessed their condition as "improved' during evaluation of the dynamics of subjective indicators. 76.5% of the main group patients no longer complained of pollakiuria (frequent urination), they also no longer had nocturnal urinary urgency. 13.2% of the main group patients reported significantly decreased pollakiuria (7 - 8 times per 24 hrs.), and nocturnal urination was reduced 2 - 3 times.

The urodynamic indicators following the administration of the pharmaceutical composition in suppository form to older and elderly patients suffering from dyuric disorders is shown in the Table.

**Table**

| ***Effect of the suppositories on the urodynamic of older and elderly patients with dysuric disorders*** | | | |
|---|---|---|---|
| Indicator | Before treatment | After treatment with traditional drugs | After treatment with the suppositories |
| Urine retention time (min) | 5.4 ± 0.7 | 5.1 ± 0.6 | 2.9 ± 0.7*# |
| Number of urinations | 9.8 ± 0.7 | 8.7 ± 0.8 | 6.5 ± 0.4*# |
| - in the daytime | 5.8± 0.6 | 5.5 ± 0.5 | 1.4 ± 0.1*# |
| - at night | | | |
| Mean urine flow rate (ml/sec) | 11.6 ± 1.5 | 12.7 ± 1.1 | 16.9 ± 1.6*# |
| Peak urine flow rate (ml/sec) | 14.2 ± 1.8 | 16.3_± 1.7 | 21.8 ± 1.9*# |
| Time to reach peak urine flow rate (ml/sec) | 7.4_± 0.1 | 6.7_± 0.6 | 5.1_± 0.9* |

| | | | |
|---|---|---|---|
| ** - P < 0. 05 in comparison to the indicator before treatment;* *# - P* < *0.05 in comparison to the indicator in the control group patients* | | | |

It is noteworthy that the control group patients did not show any positive dynamics of urinary bladder function based on both subjective and objective indicators, despite the use of traditional medications.

The uroflowmetric index in the control group patients receiving traditional therapy was practically unchanged after treatment.

The uroflowmetric index increased almost twofold in the main group patients receiving the claimed suppositories, which confirms their positive effect on urination.

After treating older and elderly patients suffering from moderate dysuric disorders with the suppositories, the recorded uroflowgrams showed restoration of the main urinary parameters to their normal values.

Elderly patients with severe dysuric disorders did not achieve fully normalized urination parameters due to the age-related lowered elasticity of the urinary bladder neck; however, such patients receiving the suppositories showed significant improvements in the strength of the urination stream and decreased need to urinate during the day (down to 7 - 8 times) and at night (down to 2 - 3 times.)

These data confirm the therapeutic efficacy of rectally administered suppositories over a 10 - 15 day period, depending on the severity of the disorder, and makes them useful for treating patients with dysuric disorders of different origins in order to restore functional activity of the detrusor-sphincter system of the urinary bladder.

### Example 6. Efficacy of the suppositories in treating genitourinary disorders in perimenopausal and postmenopausal women

24 perimenopausal and postmenopausal women aged 46 - 55 suffering from genitourinary disorders were treated with the suppositories. The women complained of hot flashes to the head and upper body, hyperhidrosis, high irritability, and emotional lability. Vaginal atrophy symptoms (vaginal dryness and itching, dyspareunia, recurrent vaginal discharge) and cystourethral atrophy symptoms (frequent daytime (pollakiuria) and nighttime (nocturia) urination, cystalgia, and urinary urgency were also reported.

The claimed suppositories were used vaginally at bedtime for 25 - 30 days.

The suppositories were found to stop subjective manifestations of vaginal (vaginal dryness and itching, dyspareunia, bleeding, etc.) and cystourethral (pollakiuria, nocturia, urinary urgency, etc.) atrophy in 83% of the women. Said symptoms disappeared both in perimenopausal and postmenopausal women. Vaginal health index for these women increased to 4 - 5 points, vaginal epithelial maturation went up to 57 - 79% (intermediate and surface cells appeared), the colposcopic picture normalized.

The symptoms of urinary stress incontinence completely disappeared in 27% perimenopausal women receiving monotherapy with the suppositories.

For other forms of dysuric disorders, therapy with the suppositories combined with hormone replacement therapy, behavior therapy, and M-cholinolytics, selective serotonin reuptake inhibitors) were used from the start. After such treatment, 82% of the patients showed positive results: daytime and nocturnal urination frequency decreased 2.5 times and 93% of the patients no longer experienced urge urinary incontinence.

Thus, the obtained results confirm the normalizing effect of the suppositories on the genitourinary tract structures, such as improved trophism and epithelium proliferation as well as normalizing detrusor overactivity due to the improved blood supply.

The claimed suppositories are useful as therapeutic and preventive agents in combination with any nosotropic therapy used for the treatment of genitourinary disorders in perimenopausal and postmenopausal women.

These studies showed that the claimed pharmaceutical composition in suppository form was effective not only for the treatment of prostate diseases (as well as the mixture of bovine prostate bioregulatory peptides) but also in treating other diseases of small pelvic organs in both men and women.

### Industrial Applicability

The claimed pharmaceutical composition can be manufactured in any pharmaceutical facility equipped for the production of suppositories.

## Claims

1. A pharmaceutical composition for use in the treatment of diseases of the lower genitourinary system in suppository form comprising a bioactive compound and a fatty base, wherein the said bioactive compound of the said composition is a mixture of amino acids: glutamic acid, lysine, alanine, arginine and glycine, and further comprising zinc in the form of zinc chloride in the following component ratio expressed in grams per one suppository weighing 2.7 - 2.9 g each:
| | |
|---|---|
| Glutamic acid | 0.050-0.500 |
| Lysine | 0.025-0.250 |
| Alanine | 0.025-0.250 |
| Arginine | 0.025-0.250 |
| Glycine | 0.025-0.250 |
| Zinc | 0.010-0.150 |
| Fatty base | remaining |

2. The pharmaceutical composition according to claim 1, wherein said composition further comprises 10 - 30 mcg of selenium in the form of anhydrous sodium selenite.

3. The pharmaceutical composition according to claim 1, wherein said composition further comprises 0.020 - 0.035 g of sodium fumarate.

## Patentansprüche

1. Pharmazeutische Zusammensetzung zur Verwendung bei der Behandlung von Krankheiten des unteren Urogenitalsystems in Suppositorienform, umfassend eine bioaktive Verbindung und eine Fettbasis, wobei die bioaktive Verbindung der Zusammensetzung eine Mischung von Aminosäuren ist: Glutaminsäure, Lysin, Alanin, Arginin und Glycin, und ferner umfassend Zink in Form von Zinkchlorid im folgenden Komponentenverhältnis, das in Gramm pro Suppositorium mit einem Gewicht von jeweils 2,7 bis 2,9 g ausgedrückt ist:
| | |
|---|---|
| Glutaminsäure | 0,050 bis 0,500 |
| Lysin | 0,025 bis 0,250 |
| Alanin | 0,025 bis 0,250 |
| Arginin | 0,025 bis 0,250 |
| Glycin | 0,025 bis 0,250 |
| Zink | 0,010 bis 0,150 |
| Fettbasis | Rest |

2. Pharmazeutische Zusammensetzung nach Anspruch 1, wobei die Zusammensetzung ferner 10 bis 30 µg Selen in Form von wasserfreiem Natriumselenit umfasst.

3. Pharmazeutische Zusammensetzung nach Anspruch 1, wobei die Zusammensetzung ferner 0,020 bis 0,035 g Natriumfumarat umfasst.

## Revendications

1. Composition pharmaceutique pour utilisation dans le traitement des maladies du système génito-urinaire inférieur sous forme de suppositoire comprenant un composé bioactif et une base grasse, dans laquelle ledit composé bioactif de ladite composition est un mélange d'acides aminés : acide glutamique, lysine, alanine, arginine et glycine, et comprenant en outre du zinc sous la forme de chlorure de zinc dans le rapport de composants suivant exprimé en grammes pour un suppositoire pesant de 2,7 à 2,9 g chacun :
| | |
|---|---|
| Acide glutamique | 0,050 - 0,500 |
| Lysine | 0,025 - 0,250 |
| Alanine | 0,025 - 0,250 |
| Arginine | 0,025 - 0,250 |
| Glycine | 0,025 - 0,250 |
| Zinc | 0,010 - 0,150 |
| Base grasse | reste |

2. Composition pharmaceutique selon la revendication 1, ladite composition comprenant en outre de 10 à 30 µg d'ions sélénium sous la forme de sélénite de sodium anhydre.

3. Composition pharmaceutique selon la revendication 1, ladite composition comprenant en outre de 0,020 à 0,035 g de fumarate de sodium.
